# EUROPEAN PATENT SPECIFICATION

(11) **EP 3 548 014 B1**
(45) Date of publication and mention of the grant of the patent: **31.07.2024**
(21) Application number: 17875201.0
(22) Date of filing: 30.11.2017
(51) Int. Cl.: A61K 31/122, A61K 31/135, A61K 31/137, C07C 25/06, C07C 225/20, A61P 15/00, A61K 9/00

(54) **KETAMINE FOR THE TREATMENT OF MENSTRUALLY RELATED SYMPTOMS**
KETAMIN ZUR BEHANDLUNG VON SYMPTOMEN IM ZUSAMMENHANG MIT DER MENSTRUATION
KÉTAMINE POUR LE TRAITEMENT DE SYMPTÔMES LIÉS AUX MENSTRUATIONS

(30) Priority: 30.11.2016 US 201662427814 P; 05.10.2017 US 201762568488 P
(43) Date of publication of application: 09.10.2019
(73) Proprietor: Wolfson, Philip, E., San Anselmo, CA 94960 (US)
(72) Inventor: Wolfson, Philip, E., San Anselmo, CA 94960 (US)
(74) Representative: Kransell & Wennborg KB
(86) International application number: PCT/US2017/063836
(87) International publication number: WO 2018/102488

(56) References cited:
- WO-A1-2017/165877
- US-A1- 2014 256 821
- US-A1- 2015 231 129
- US-A1- 2015 313 892
- BORN L ET AL: "CURRENT MANAGEMENT OF PREMENSTRUAL SYNDROME AND PREMENSTRUAL DYSPHORIC DISORDER", CURRENT PSYCHIATRY REPORTS, CURRENT SCIENCE, US, vol. 3, no. 6, 1 December 2001 (2001-12-01), pages 463 - 469, XP009021168, ISSN: 1523-3812
- BAKER FC ET AL.: "Daytime Sleepiness, Psychomotor Performance, Waking EEG Spectra and Evoked Potentials in Women with Severe Premenstrual Syndrome", J. SLEEP RES., vol. 19, 2010, pages 214 - 227;1-27, XP055490390

## Description

### TECHNICAL FIELD

The present disclosure relates to ketamine or a pharmaceutically acceptable salt thereof, for use in a method of treating a menstrually related symptom in a subject in need thereof.

### BACKGROUND

The present disclosure features methods, compositions, and kits for the treatment of all forms of emotional and painful symptoms related to the menstrual cycle, including symptoms characteristic of menstrual cycle-related disorders, such as premenstrual dysphoric disorder (PMDD), premenstrual syndrome (PMS), menopause, endometriosis, perimenopause, and sub-diagnostic emotional, psychological, cognitive, spiritual, and/or physical symptoms of the menstrual cycle.

Emotional and physical responses to the menstrual cycle range from mild to profound and may last from hours to many days affecting the quality of life, well-being, relationships, and an experience of illness and emotional or physical alteration for a majority of women. The more severe forms (PMDD affects 3-8 % of women and PMS affects 20-32%) and lesser symptoms bring discomfort to myriad women. Perimenopause brings forth its own set of difficulties with often more profound menstrual cycles and attendant emotional or physical difficulties. Menopause may include cognitive and emotional changes that lead to feelings of irritability, emotional sensitivity, diminished memory, intellectual impairment and diminished well-being.

Overall these menstrual cycle and menopause related difficulties may include dysphoria, diminished interest or pleasure in activities, decrease or increase in appetite, troubled sleep or hypersomnia, insomnia, psychomotor agitation or retardation, fatigue or loss of energy, irritability, feelings of worthlessness or excessive or inappropriate guilt, diminished ability to think or concentrate or indecisiveness, anhedonia, anxiety, and in more severe states recurrent thoughts of death, suicidal ideation or suicidal attempts and anger and aggressive feelings and behavior. A variety of somatic symptoms may also be present. These include varying degrees of cramping, fatigue, back pain, breast tenderness, headaches, and other manifestations. Underlying emotional and physical problems may be exacerbated including dysphoria, anxiety, and Post-Traumatic Stress Disorder. Difficulties between partners, with children, relatives and friends may become heightened and can cause alienation, fracturing and even separation and divorce. Workplace difficulties may occur or become exacerbated including tardiness, absences, and conflicts with co-workers and with authority. The consequences of these menstrually related difficulties include costs for treatment, loss of wages, loss of productivity, and other social costs.

Ketamine's many decades long use has established its safety. In the claimed use, dosages are much lower than that used for anesthesia and side effects are minimal.

US 2015/313892 discloses pharmaceutical compositions for treating pain associated with dysmenorrhea, such as a synergistically acting sub-therapeutic combination of a nontoxic N-methyl-D-aspartate receptor antagonist such as dextromethorphan, magnesium, dextrorphan, ketamine or pharmaceutically acceptable salt thereof, tramadol or its analog such as recemic tramadol or an analogously acting molecular entity or pharmaceutically acceptable salt thereof, and an anticonvulsant and/or a tricyclic anti-depressant or pharmaceutically acceptable salt thereof.

US 2014/256821 suggests intranasal administration of ketamine to treat depression.

US 2015/231129 relates to methods, compositions, and kits for treating or preventing symptoms of hormonal variation. The method comprises the steps of administering an effective amount of Dextromethorphan or Dextrorphan, or a pharmaceutically acceptable salt thereof, to a subject having one or more symptoms of hormonal variations.

WO 2017/1656877 relates to the use of (2R, 6R)-hydroxynorketamine and (2S, 6S)-hydroxynorketamine in the treatment of depression, anxiety, anhedonia, suicidal ideation and post-traumatic stress disorders.

That women with severe PMS are sleepy and fatigued is discussed in Baker F.C., et al., Daytime Sleepiness, Psychomotor Performance, Waking EEG Spectra and Evoked Potentials in Women with Severe Premenstrual Syndrome, J. Sleep Res., Vol. 19, 2010, pages 214-227.

Applicants have discovered that ketamine can be useful for ameliorating menstrually related symptoms, including symptoms characteristic of menstrual cycle-related disorders, such as PMDD and PMS.

### SUMMARY

The invention features ketamine or a pharmaceutically acceptable salt thereof, for use in a method of treating a menstrually related symptom in a subject in need thereof, as defined in independent claim 1. The method including administering to the subject ketamine or a pharmaceutically acceptable salt thereof, in an amount sufficient to treat the menstrually related symptom. The menstrually related symptom can be selected from mood swings, dysphoria, negativism, diminished mental capabilities, anxiety, irritability, and anger. In some embodiments, not claimed, the menstrually related symptom is hypersomnia, insomnia, difficulty in concentration, or lethargy. In particular embodiments, the menstrually related symptom is caused by premenstrual syndrome (PMS) or premenstrual dysphoric disorder (PMDD). In one particular embodiment, not claimed, the menstrually related symptom is refractory to treatment with NSAIDs, corticosteroids, muscle relaxants, or antidepressants.

In one embodiment, not claimed, the method can include initiating treatment following the appearance of a menstrually related symptom in the subject.

In another embodiment, not claimed, the method can include initiating treatment prior to the appearance of a menstrually related symptom in the subject (e.g., in anticipation or expectation of a symptom based upon the timing of the subject's menstrual cycle and past experiences with the cyclical appearance of symptoms).

In any of the above methods the ketamine, or a pharmaceutically acceptable salt thereof, is administered once or more daily, once or more every other day, or once or more every three days, or depending on the presence and/or severity of menstrually related symptoms. For example, the can be once or twice daily for a period of from 1 to 10 days (e.g., for a period of from 2 to 8 days, from 2 to 7 days, from 2 to 6 days, or for a period of 2 days, 3 days, 4 days, 5 days, 6 days, 7 days, 8 days, 9 days, or 10 days), followed by a period of at least one or two weeks during which no ketamine, or a pharmaceutically acceptable salt thereof, is administered to the subject. In particular embodiments, the administration is once or more daily, or intermittently for a period of from 1 to 8 days, followed by a period of at least one or two weeks during which no ketamine, or a pharmaceutically acceptable salt thereof, is administered to the subject. In particular embodiments, the ketamine, or a pharmaceutically acceptable salt thereof, is administered to the subject only in the evening. In particular embodiments, the ketamine, or a pharmaceutically acceptable salt thereof, is administered to the subject once or more daily in the evening. In particular embodiments, the ketamine, or a pharmaceutically acceptable salt thereof, is administered to the subject during the day time one or more times. In particular embodiments, the ketamine, or a pharmaceutically acceptable salt thereof, is administered to the subject one or more times daily and/or in the evening.

In particular embodiments of any of the above methods, the method includes administering an average daily dose of from 1 mg to 500 mg (e.g., 10 mg to 200 mg, 25 mg to 150 mg, or 35 mg to 125 mg) of ketamine, or a pharmaceutically acceptable salt thereof, to the subject. For example, the method can include administering an average daily dose of from 10 mg to 200 mg (e.g., 30 ± 20 mg, 60 ± 20 mg, 90 ± 20 mg, or 150 ± 50 mg) of enantiomerically pure S-(+)-ketamine, or a pharmaceutically acceptable salt thereof, to the subject. In particular embodiments, the method includes administering an average daily dose of from 10 mg to 200 mg (e.g., 30 ± 20 mg, 60 ± 20 mg, 90 ± 20 mg, or 150 ± 50 mg) of enantiomerically pure R-(-)-ketamine, or a pharmaceutically acceptable salt thereof, to the subject. In some embodiments, the method includes administering an average daily dose of from 10 mg to 200 mg (e.g., 30 ± 20 mg, 60 ± 20 mg, 90 ± 20 mg, or 150 ± 50 mg) of racemic ketamine, or a pharmaceutically acceptable salt thereof, to the subject.

In particular embodiments of any of the above methods, the administration is by a route selected from oral, sublingual, intranasal, intramuscular, intravenous, transdermal, and rectal administration, or any administration route described herein.

In particular embodiments of any of the above methods, the ketamine, or a pharmaceutically acceptable salt thereof, is administered in an amount, or in a dosage form (e.g., a sustained release dosage form), that, upon administration to the subject, is sub-anesthetic (i.e., produces effects on consciousness and cognition equivalent to less than those effects observed with 2.0 mg/kg of racemic ketamine administered intravenously).

In some embodiments, not claimed, the method further includes concurrently administering to the subject a muscle relaxant. The muscle relaxant can be selected from afloqualone, baclofen, carisoprodol, chlormezanone, chlorphenesin carbamate, chlorzoxasozone, cyclobenzaprine, clonazepam, dantrolene, diazepam, eperisone, idrocilamide, inaperisone, mephenesin, mephenoxalone, methocarbamol, metaxalone, mivacurium chloride, orphenadrine, phenprobamate, pridinol mesylate, quinine, tetrazepam, thiocolchicoside, tizanidine, tolperisone, and pharmaceutically acceptable salts thereof.

In some embodiments, not claimed, the method further includes concurrently administering to the subject an anti-inflammatory agent, such as NSAIDs, including ibuprofen in its various forms and naproxen in its various forms, and corticosteroids.

In some embodiments, not claimed, the method further includes concurrently administering to the subject fluoxetine, or other anti-depressants described herein.

As used herein, the term "average daily dose" refers to the average amount of ketamine, or a pharmaceutically acceptable salt thereof, administered to a subject for a given dosing regimen. For example, single doses of 100 mg of ketamine administered every other day is an average daily dose of 50 mg. For a regimen in which 15 mg doses of ketamine are administered twice daily, the average daily dose is 30 mg.

By "corticosteroid" is meant any naturally occurring or synthetic compound characterized by a hydrogenated cyclopentanoperhydrophenanthrene ring system. Naturally occurring corticosteroids are generally produced by the adrenal cortex. Synthetic corticosteroids may be halogenated. Exemplary corticosteroids are described herein.

As used herein, the term "initiating treatment" refers to commencing treatment in a subject that has not received ketamine, or a pharmaceutically acceptable salt thereof, for at least 3 days, 5 days, 7 days, 10 days, or 14 days.

As used herein, the term "menstrually related symptom" refers to an intermittent symptom experienced by a subject that change in frequency and/or intensity as a function of the subject's menstrual cycle. The symptoms typically include one or more of dysphoria, mood swings, anxiety, marked anger, irritability, tension, decreased interest in usual activities, fatigue, change in appetite, sleep problems, hypersomnia, difficulty in concentration, lethargy, and physical problems, such as headache, cramps, back pain, joint pain, and breast tenderness. For some subjects, the menstrually related symptom is the result of, or part of the diagnosis of, PMS or PMDD.

As used herein, the term "pharmaceutically acceptable salts" refers to salts of the active compounds of the invention that can be prepared in situ during the final isolation and purification of the compounds of the invention, or separately by reacting the free base function with a suitable organic or inorganic acid. Representative acid addition salts include acetate, adipate, alginate, ascorbate, aspartate, benzenesulfonate, benzoate, bisulfate, camphorate, camphersulfonate, citrate, ethanesulfonate, hydrobromide, hydrochloride, hydroiodide, 2-hydroxy-ethanesulfonate, lactate, malate, maleate, malonate, mesylate, methanesulfonate, oxalate, succinate, sulfate, and tartrate salts.

A "therapeutically effective amount" or "an amount sufficient" of a drug is an amount effective to demonstrate a desired activity of the drug. According to the instant invention, a therapeutically effective amount of ketamine is an amount effective to alleviate, i.e., noticeably reduce, the symptoms of menstrually related symptoms.

As used herein, the term "treating" refers to administering a pharmaceutical composition for therapeutic purposes. To "treat disease" or use for "therapeutic treatment" refers to administering treatment to a subject already suffering from a condition to improve or stabilize the subject's condition. For example, treating includes ameliorating a menstrually related symptom in a subject by dosing the subject just prior to, during, or after the subject is exhibiting the menstrually related symptom. Treating also includes ameliorating one or more symptoms of menopause or endometriosis in a subject by dosing the subject prior to, during, or after the subject is exhibiting the symptom.

### DETAILED DESCRIPTION

The invention for the first time provides ketamine, or a pharmaceutically acceptable salt thereof, for use in a method of treating menstrually related symptoms. The compositions of the invention may be designed to be short-acting, fast-releasing, long-acting, or sustained-releasing as described herein. Thus, the pharmaceutical formulations may also be formulated for controlled release or for slow release.

Ketamine is an inexpensive, readily available drug, with minor adverse side effects. Thus, the invention contemplates additional savings to the overburdened health care system. Sublingual administration of this agent is rapid, allowing for fast action of the drug, and easily accomplished by a non-medically trained patient.

Contemplated herein is a package comprising a carrier for delivering a sublingual lozenge (troche) or oral dissolving tablets containing ketamine. Oral (buccal, sublingual) mucosal absorption of ketamine is a reliable method for administering ketamine that poses little hazard for abuse. Using sub-anesthetic dosages at specific levels designed to provide relief of menstrual cycle related symptoms is the goal of this and any other methods, such as the possibility of an intranasal formulation for administering ketamine on its own or coupled with other agents for this indication.

A further advantage of the invention is that the patient can administer ketamine on an as needed, dose-to-effect basis. Thus, the frequency of administration is under control of the patient. However, the relatively low dose with each administration and the sublingual route of administration will reduce the possibilities for abuse, especially since it is difficult to use multiple lozenges at the same time. Yet another particular advantage of the present invention is that sublingual administration of ketamine is noninvasive, and provides for rapid introduction of effect within minutes. Control of the frequency and quantity of prescription is entirely under the physician's medical judgement.

As discussed above, the present disclosure relates to various methods and compositions for treating menstrually related symptoms. An alternative route of administration could comprise intranasal administration of ketamine. While the sublingual route is preferred because of its lower possibility for abuse, an intranasal preparation for this method may be contemplated with appropriate safeguards for administration. Such treatment may be administered alone or may be supplemented with other therapies as described herein.

### Ketamine and Norketamine

This relates to ketamine, or a pharmaceutically acceptable salt thereof, for use in a method of treating a menstrually related symptom in a subject in need threreof, wherein said menstrually related symptom is selected from mood swings, dysphoria, negativism, diminished mental capabilities, anxiety, irritability, and anger, as defined in claim 1.

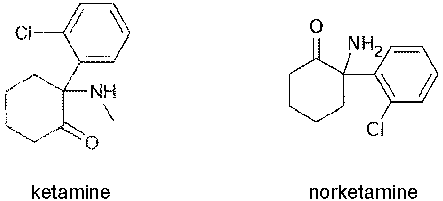

As used herein, the term "ketamine" includes ketamine in its racemic (R/S) form, in its R-(-) enantiomerically pure form, or in its S-(+) enantiomerically pure form.

As used herein, "enantiomerically pure" refers to compositions consisting substantially of a single isomer (i.e., substantially free of the opposite isomer), preferably consisting of 90%, 92%, 95%, 98%, 99%, or 100% (w/w) of a single isomer. For example, when the methods of the invention include the administration of enantiomerically pure R-(-)-ketamine, the pharmaceutical composition administered can include at least 95% (w/w) S-(+)-ketamine, and less than 5% (w/w) R-(-)-ketamine.

Ketamine racemate is primarily used for the induction and maintenance of general anesthesia. Enantiomerically pure S-(+)-ketamine (aka esketamine) is available for medical use, administered either IV (intravenously) or IM (intramuscularly), under the brand name KETANEST^{®}. Enantiomerically pure R-(-)-ketamine is also known as arketamine. Ketamine is converted metabolically through demethylation to norketamine, in vivo, at rates dependent on the route of administration. For use in anesthesia, S-(+)-ketamine has been reported to be twice as potent as R-(-)-ketamine, and norketamine has been reported to have one third the potency of ketamine (C. S. T. Aun, Br. J. Anaesthesia 83: 29-41 (1999)).

As used herein, the term "6-hydroxynorketamine" includes 6-hydroxynorketamine in any of its 2R,6R; 2S,6S; 2S,6R; and 2R,6S isomerically pure forms (shown below).

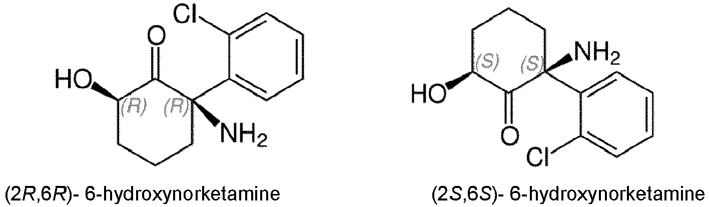

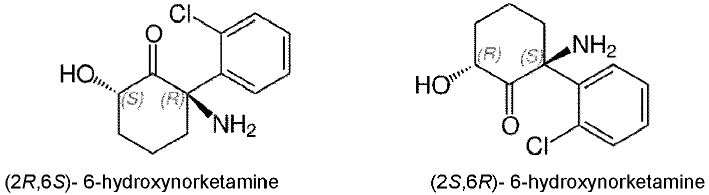

As used herein, "isomerically pure" refers to compositions consisting substantially of a single diastereomer (i.e., substantially free of other isomers), preferably consisting of 90%, 92%, 95%, 98%, 99%, or 100% (w/w) of a single isomer.

### Formulation of Pharmaceutical Compositions

The administration of ketamine, or a pharmaceutically acceptable salt thereof, (the active compound) may be by any suitable means that results in relief of a menstrually related symptom. The active may be contained in any appropriate amount in any suitable carrier substance, and is generally present in an amount of 1-95% by weight of the total weight of the composition. The composition may be provided in a dosage form that is suitable for the sublingual, buccal, oral, parenteral (e.g., intravenously, intramuscularly), intranasal, transdermal, or rectal administration route. Thus, the composition may be in the form of, e.g., tablets, capsules, pills, powders, granulates, suspensions, emulsions, solutions, gels including hydrogels, pastes, ointments, creams, plasters, drenches, osmotic delivery devices, suppositories, enemas, injectables, sprays, or aerosols. The pharmaceutical compositions may be formulated according to conventional pharmaceutical practice (see, e.g., Remington: The Science and Practice of Pharmacy (20th ed.), ed. A.R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Pharmaceutical compositions may be formulated to release the active compound substantially immediately upon administration or at any predetermined time or time period after administration. The latter types of compositions are generally known as controlled release formulations, which include (i) formulations that create a substantially constant concentration of the active compound within the body over an extended period of time; (ii) formulations that after a predetermined lag time create a substantially constant concentration of the active compound within the body over an extended period of time; and (iii) formulations that sustain active compound action during a predetermined time period by maintaining a relatively, constant, effective active compound level in the body with concomitant minimization of undesirable side effects associated with fluctuations in the plasma level of the active compound (sawtooth kinetic pattern).

Administration of the active compound in the form of a controlled release formulation is especially preferred in cases in which the active compound, either alone or in combination with a second agent, at therapeutic levels produces unwanted side effects, such as nausea.

Any of a number of strategies can be pursued in order to obtain controlled release of the active compound in question. In one example, controlled release is obtained by appropriate selection of various formulation parameters and ingredients, including, e.g., various types of controlled release compositions and coatings. Thus, the drug is formulated with appropriate excipients into a pharmaceutical composition that, upon administration, releases the active compound in a controlled manner. Examples include single or multiple unit tablet or capsule compositions, oil solutions, suspensions, emulsions, microcapsules, microspheres, nanoparticles, patches, and liposomes.

### Sublingual and buccal dosage forms

Formulations for sublingual may be in the form of films, strips, lozenges, and orally dissolving tablets. An orally dissolving tablet (ODT) refers to pharmaceutical dosage form designed to be dissolved on the tongue rather than swallowed whole, or designed to dissolve on the sublingual or buccal mucosa for sublingual or mucosal administration. Alternatively, the dosage form can be a lozenge (for slower administration as the lozenge dissolved over the course of 5-10 minutes), or as a rapidly dissolving film (dissolving over the course of less than 2 minutes). The active compound is administered via absorption in the mouth (i.e., buccally or sublingually). The formulation excipients are edible and pharmaceutically acceptable using excipients known in the art for the preparation of films, strips, lozenges, and orally dissolving tablets. For example, a film is prepared typically using hydrophilic polymers that rapidly dissolves on the tongue, palatine tissue, or buccal cavity, delivering the active compound to the systemic circulation via dissolution when contact with liquid is made.

### Solid dosage forms for oral use

Formulations for oral use include tablets containing the active ingredient(s) in a mixture with nontoxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

The tablets may be uncoated or they may be coated by known techniques, optionally to delay disintegration and absorption in the gastrointestinal tract and thereby providing a sustained action over a longer period. The coating may be adapted to release the active compound in a predetermined pattern (e.g., in order to achieve a controlled release formulation) or it may be adapted not to release the active compound until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material such as, e.g., glyceryl monostearate or glyceryl distearate may be employed.

The solid tablet compositions may include a coating adapted to protect the composition from unwanted chemical changes, (e.g., chemical degradation prior to the release of the active compound). The coating may be applied on the solid dosage form in a similar manner as that described in Encyclopedia of Pharmaceutical Technology, supra.

For combination therapies, two drugs may be mixed together in the tablet, or may be partitioned. In one example, the first drug is contained on the inside of the tablet, and the second drug is on the outside, such that a substantial portion of the second drug is released prior to the release of the first drug.

Formulations for oral use may also be presented as chewable tablets, or as hard gelatin capsules wherein the active compound is mixed with an inert solid diluent (e.g., potato starch, lactose, microcrystalline cellulose, calcium carbonate, calcium phosphate or kaolin), or as soft gelatin capsules wherein the active compound is mixed with water or an oil medium, for example, peanut oil, liquid paraffin, or olive oil. Powders and granulates may be prepared using the ingredients mentioned above under tablets and capsules in a conventional manner using, e.g., a mixer, a fluid bed apparatus or a spray drying equipment.

### Controlled release oral dosage forms

Controlled release compositions for oral use may, e.g., be constructed to release the active compound by controlling the dissolution and/or the diffusion of the active drug substance.

Dissolution or diffusion controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dlpolylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more of the compounds of the claimed combinations may also be in the form of a buoyant tablet or capsule (i.e., a tablet or capsule that, upon oral administration, floats on top of the gastric content for a certain period of time). A buoyant tablet formulation of the compound(s) can be prepared by granulating a mixture of the drug(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

### Liquids for oral administration

Powders, dispersible powders, or granules suitable for preparation of an aqueous suspension by addition of water are convenient dosage forms for oral administration. Formulation as a suspension provides the active compound in a mixture with a dispersing or wetting agent, suspending agent, and one or more preservatives. Suitable dispersing or wetting agents are, for example, naturally-occurring phosphatides (e.g., lecithin or condensation products of ethylene oxide with a fatty acid, a long chain aliphatic alcohol, or a partial ester derived from fatty acids) and a hexitol or a hexitol anhydride (e.g., polyoxyethylene stearate, polyoxyethylene sorbitol monooleate, polyoxyethylene sorbitan monooleate, and the like). Suitable suspending agents are, for example, sodium carboxymethylcellulose, methylcellulose, sodium alginate, and the like.

### Parenteral compositions

The pharmaceutical composition may also be administered parenterally by injection, infusion or implantation (intravenous, intramuscular, subcutaneous, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation. Formulations can be found in Remington: The Science and Practice of Pharmacy, supra.

Compositions for parenteral use may be provided in unit dosage forms (e.g., in single-dose ampoules), or in vials containing several doses and in which a suitable preservative may be added (see below). The composition may be in form of a solution, a suspension, an emulsion, an infusion device, or a delivery device for implantation, or it may be presented as a dry powder to be reconstituted with water or another suitable vehicle before use. Apart from the active compound, the composition may include suitable parenterally acceptable carriers and/or excipients. The active drug(s) may be incorporated into microspheres, microcapsules, nanoparticles, liposomes, or the like for controlled release. Furthermore, the composition may include suspending, solubilizing, stabilizing, pH-adjusting agents, and/or dispersing agents.

As indicated above, the pharmaceutical compositions may be in the form suitable for sterile injection. To prepare such a composition, the suitable active compound is dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1 ,3-butanediol, Ringer's solution, and isotonic sodium chloride solution. The aqueous formulation may also contain one or more preservatives (e.g., methyl, ethyl or n-propyl p-hydroxybenzoate).

### Controlled release parenteral compositions

Controlled release parenteral compositions may be in form of aqueous suspensions, microspheres, microcapsules, magnetic microspheres, oil solutions, oil suspensions, or emulsions. Alternatively, the active drug(s) may be incorporated in biocompatible carriers, liposomes, nanoparticles, implants, or infusion devices.

Materials for use in the preparation of microspheres and/or microcapsules are, e.g., biodegradable/bioerodible polymers such as polygalactin, poly-(isobutyl cyanoacrylate), poly(2-hydroxyethyl-L-glutamnine) and, poly(lactic acid). Biocompatible carriers that may be used when formulating a controlled release parenteral formulation are carbohydrates (e.g., dextrans), proteins (e.g., albumin), lipoproteins, or antibodies. Materials for use in implants can be non-biodegradable (e.g., polydimethyl siloxane) or biodegradable (e.g., poly(caprolactone), poly(lactic acid), poly(glycolic acid) or poly(ortho esters)).

### Rectal compositions

For rectal application, suitable dosage forms for a composition include suppositories (emulsion or suspension type), and rectal gelatin capsules (solutions or suspensions). In a typical suppository formulation, the active compound is combined with an appropriate pharmaceutically acceptable suppository base such as cocoa butter, esterified fatty acids, glycerinated gelatin, and various watersoluble or dispersible bases like polyethylene glycols and polvoxyethylene sorbitan fatty acid esters. Various additives, enhancers, or surfactants may be incorporated.

### Intranasal and Inhalation Compositions

For administration by inhalation, typical dosage forms include nasal sprays and aerosols. In a typically nasal formulation, the active compound is dissolved or dispersed in a suitable vehicle. The pharmaceutically acceptable vehicles and excipients (as well as other pharmaceutically acceptable materials present in the composition such as diluents, enhancers, flavoring agents, and preservatives) are selected in accordance with conventional pharmaceutical practice in a manner understood by the persons skilled in the art of formulating pharmaceuticals.

### Percutaneous and topical compositions

The pharmaceutical compositions may also be administered topically on the skin for percutaneous absorption in dosage forms or formulations containing conventionally non-toxic pharmaceutical acceptable carriers and excipients including microspheres and liposomes. The formulations include creams, ointments, lotions, liniments, gels, hydrogels, solutions, suspensions, sticks, sprays, pastes, plasters, and other kinds of transdermal drug delivery systems. The pharmaceutically acceptable carriers or excipients may include emulsifying agents, antioxidants, buffering agents, preservatives, humectants, penetration enhancers, chelating agents, gel-forming agents, ointment bases, perfumes, and skin protective agents.

Examples of emulsifying agents are naturally occurring gums (e.g., gum acacia or gum tragacanth) and naturally occurring phosphatides (e.g., soybean lecithin and sorbitan monooleate derivatives). Examples of antioxidants are butylated hydroxy anisole (BHA), ascorbic acid and derivatives thereof, tocopherol and derivatives thereof, butylated hydroxy anisole, and cysteine. Examples of preservatives are parabens, such as methyl or propyl p-hydroxybenzoate, and benzalkonium chloride. Examples of humectants are glycerin, propylene glycol, sorbitol, and urea. Examples of penetration enhancers are propylene glycol, DMSO, triethanolamine, N,N-dimethylacetamide, N,N-dimethylformamide, 2-pyrrolidone and derivatives thereof, tetrahydrofurfuryl alcohol, and AZONETM. Examples of chelating agents are sodium EDTA, citric acid, and phosphoric acid. Examples of gel forming agents are CARBOPOLTM, cellulose derivatives, bentonite, alginates, gelatin and polyvinylpyrrolidone. Examples of ointment bases are beeswax, paraffin, cetyl palmitate, vegetable oils, sorbitan esters of fatty acids (Span), polyethylene glycols, and condensation products between sorbitan esters of fatty acids and ethylene oxide (e.g., polyoxyethylene sorbitan monooleate (TWEENTM)).

The compositions may be adapted for direct application or for introduction into relevant orifice(s) of the body (e.g., rectal, urethral, vaginal or oral orifices). The composition may be applied by means of special drug delivery devices such as dressings or alternatively plasters, pads, sponges, strips, or other forms of suitable flexible material.

### Dosages

The dosage of ketamine, or a pharmaceutically acceptable salt thereof, to be administered depends on several factors, including: the administration method, the condition or symptom to be treated, the severity of the condition or symptom, whether the condition is to be treated or prevented, and the age, weight, and health of the person to be treated. Additionally, pharmacogenomic (the effect of genotype on the pharmacokinetic, pharmacodynamic or efficacy profile of a therapeutic) information about a particular patient may affect dosage used.

As described above, the active compound may be administered orally in the form of tablets, capsules, elixirs or syrups, or rectally in the form of suppositories. Parenteral administration of the active compound is suitably performed, for example, in the form of saline solutions or with the compound incorporated into liposomes. Sublingual or buccal administration of the active compound may be in the form of films, strips, lozenges, and orally dissolving tablets.

The amount administered can be from about 0.01 mg of active compound per kg of the subject's body weight (mg/kg) to about 5 mg/kg (e.g., from about 0.05 mg/kg to about 1 mg/kg, from about 0.05 mg/kg to about 0.5 mg/kg, from about 1 mg/kg to about 3 mg/kg, or from about 2 mg/kg to about 5 mg/kg), depending upon the route of administration. In general, the dose will be in the range of about 10 mg/day to about 200 mg/day (e.g., from about 25 mg/day to about 150 mg/day or from about 50 mg/day to about 200 mg/day) for sublingual administration. The dose will be in the range of about 5 mg/day to about 100 mg/day (e.g., from about 5 mg/day to about 50 mg/day or from about 25 mg/day to about 100 mg/day) for intranasal administration. The dose will be in the range of about 2 mg/day to about 75 mg/day (e.g., from about 2 mg/day to about 35 mg/day or from about 25 mg/day to about 75 mg/day) for intravenous administration. The dose will be in the range of about 10 mg/day to about 75 mg/day (e.g., from about 10 mg/day to about 45 mg/day or from about 40 mg/day to about 75 mg/day) for intramuscular administration. The dose will be in the range of about 50 mg/day to about 250 mg/day (e.g., from about 50 mg/day to about 125 mg/day or from about 100 mg/day to about 250 mg/day) for transdermal administration.

### Therapy

Therapy may be provided at home, the doctor's office, a clinic, a hospital's outpatient department, or a hospital. Treatment generally begins at the physician's office so that the doctor can observe the therapy's effects closely and make any adjustments that are needed. The duration of the therapy depends on the type and severity of the menstrually related symptoms being treated, the age and condition of the patient, the stage and type of the patient's menstrually related condition, and how the patient responds to the treatment. Additionally, a person having a greater risk of developing menstrually related symptoms (e.g., a person who is genetically predisposed or previously had menstrually related symptoms) may receive prophylactic treatment to inhibit or delay or reduce the severity of a menstrually related symptom.

Optionally, the ketamine, or a pharmaceutically acceptable salt thereof, is administered in combination with a second agent (e.g., a muscle relaxant or an anti-inflammatory agent). For combination therapies, the dosage, frequency and mode of administration of each component of the combination can be controlled independently. For example, ketamine, or a pharmaceutically acceptable salt thereof, may be administered sublingually in a regimen described herein, while the second agent may be administered orally once per day. Combination therapy may be given in on-and-off cycles that include rest periods dictated by the patient's menstrual cycle and/or the intermittent nature of the symptoms. The combination of therapeutic agents may also be formulated together such that one administration delivers both actives.

### Muscle Relaxants

If desired, the ketamine, or a pharmaceutically acceptable salt thereof, may be administered in conjunction with one or more muscle relaxants, such as afloqualone, baclofen, carisoprodol, chlormezanone, chlorphenesin carbamate, chlorzoxasozone, cyclobenzaprine, clonazepam, dantrolene, diazepam, eperisone, idrocilamide, inaperisone, mephenesin, mephenoxalone, methocarbamol, metaxalone, mivacurium chloride, orphenadrine, phenprobamate, pridinol mesylate, quinine, tetrazepam, thiocolchicoside, tizanidine, tolperisone, and pharmaceutically acceptable salts thereof. Two or more muscle relaxants can be administered in the same treatment. This combination can be especially useful for situations in which the dominant menstrually related symptoms experienced by the subject include cramping.

### Non-Steroidal Anti-Inflammatory Drugs (NSAIDs)

If desired, the ketamine, or a pharmaceutically acceptable salt thereof, may be administered in conjunction with one or more non-steroidal anti-inflammatory drugs (NSAIDs), such as naproxen sodium, diclofenac sodium, diclofenac potassium, aspirin, sulindac, diflunisal, piroxicam, indomethacin, ibuprofen, nabumetone, choline magnesium trisalicylate, sodium salicylate, salicylsalicylic acid (salsalate), fenoprofen, flurbiprofen, ketoprofen, meclofenamate sodium, meloxicam, oxaprozin, sulindac, and tolmetin. Two or more NSAIDs can be administered in the same treatment. This combination can be especially useful for situations in which the dominant menstrually related symptom experienced by the subject is physical pain.

### Corticosteroids

If desired, the ketamine, or a pharmaceutically acceptable salt thereof, may be administered in conjunction with one or more corticosteroids. Suitable corticosteroids include 11-alpha,17-alpha,21-trihydroxypregn-4-ene-3,20-dione; 11-beta,16-alpha,17,21-tetrahydroxypregn-4-ene-3,20-dione; 11-beta,16-alpha,17,21-tetrahydroxypregn-1,4-diene-3,20-dione; 11-beta,17-alpha,21-trihydroxy-6-alpha-methylpregn-4-ene-3,20-dione; 11-dehydrocorticosterone; 11-deoxycortisol; 11-hydroxy-1,4-androstadiene-3,17-dione; 11-ketotestosterone; 14-hydroxyandrost-4-ene-3,6,17-trione; 15,17-dihydroxyprogesterone; 16-methylhydrocortisone; 17,21-dihydroxy-16-alpha-methylpregna-1,4,9(11)-triene-3,20-dione; 17-alpha-hydroxypregn-4-ene-3,20-dione; 17-alpha-hydroxypregnenolone; 17-hydroxy-16-beta-methyl-5-beta-pregn-9(11)-ene-3,20-dione; 17-hydroxy-4,6,8(14)-pregnatriene-3,20-dione; 17-hydroxypregna-4,9(11)-diene-3,20-dione; 18-hydroxycorticosterone; 18-hydroxycortisone; 18-oxocortisol; 21-deoxyaldosterone; 21-deoxycortisone; 2-deoxyecdysone; 2-methylcortisone; 3-dehydroecdysone; 4-pregnene-17-alpha,20-beta, 21-triol-3,11-dione; 6,17,20-trihydroxypregn-4-ene-3-one; 6-alpha-hydroxycortisol; 6-alpha-fluoroprednisolone, 6-alpha-methylprednisolone, 6-alpha-methylprednisolone 21-acetate, 6-alpha-methylprednisolone 21-hemisuccinate sodium salt, 6-beta-hydroxycortisol, 6-alpha, 9-alpha-difluoroprednisolone 21-acetate 17-butyrate, 6-hydroxycorticosterone; 6-hydroxydexamethasone; 6-hydroxyprednisolone; 9-fluorocortisone; alclometasone dipropionate; aldosterone; algestone; alphaderm; amadinone; amcinonide; anagestone; androstenedione; anecortave acetate; beclomethasone; beclomethasone dipropionate; beclomethasone dipropionate monohydrate; betamethasone 17-valerate; betamethasone sodium acetate; betamethasone sodium phosphate; betamethasone valerate; bolasterone; budesonide; calusterone; chlormadinone; chloroprednisone; chloroprednisone acetate; cholesterol; clobetasol; clobetasol propionate; clobetasone; clocortolone; clocortolone pivalate; clogestone; cloprednol; corticosterone; cortisol; cortisol acetate; cortisol butyrate; cortisol cypionate; cortisol octanoate; cortisol sodium phosphate; cortisol sodium succinate; cortisol valerate; cortisone; cortisone acetate; cortodoxone; daturaolone; deflazacort, 21-deoxycortisol, dehydroepiandrosterone; delmadinone; deoxycorticosterone; deprodone; descinolone; desonide; desoximethasone; dexafen; dexamethasone; dexamethasone 21-acetate; dexamethasone acetate; dexamethasone sodium phosphate; dichlorisone; diflorasone; diflorasone diacetate; diflucortolone; dihydroelatericin a; domoprednate; doxibetasol; ecdysone; ecdysterone; endrysone; enoxolone; flucinolone; fludrocortisone; fludrocortisone acetate; flugestone; flumethasone; flumethasone pivalate; flumoxonide; flunisolide; fluocinolone; fluocinolone acetonide; fluocinonide; 9-fluorocortisone; fluocortolone; fluorohydroxyandrostenedione; fluorometholone; fluorometholone acetate; fluoxymesterone; fluprednidene; fluprednisolone; flurandrenolide; fluticasone; fluticasone propionate; formebolone; formestane; formocortal; gestonorone; glyderinine; halcinonide; hyrcanoside; halometasone; halopredone; haloprogesterone; hydrocortiosone cypionate; hydrocortisone; hydrocortisone 21-butyrate; hydrocortisone aceponate; hydrocortisone acetate; hydrocortisone buteprate; hydrocortisone butyrate; hydrocortisone cypionate; hydrocortisone hemisuccinate; hydrocortisone probutate; hydrocortisone sodium phosphate; hydrocortisone sodium succinate; hydrocortisone valerate; hydroxyprogesterone; inokosterone; isoflupredone; isoflupredone acetate; isoprednidene; meclorisone; mecortolon; medrogestone; medroxyprogesterone; medrysone; megestrol; megestrol acetate; melengestrol; meprednisone; methandrostenolone; methylprednisolone; methylprednisolone aceponate; methylprednisolone acetate; methylprednisolone hemisuccinate; methylprednisolone sodium succinate; methyltestosterone; metribolone; mometasone; mometasone furoate; mometasone furoate monohydrate; nisone; nomegestrol; norgestomet; norvinisterone; oxymesterone; paramethasone; paramethasone acetate; ponasterone; prednisolamate; prednisolone; prednisolone 21-hemisuccinate; prednisolone acetate; prednisolone farnesylate; prednisolone hemisuccinate; prednisolone-21 (beta-D-glucuronide); prednisolone metasulphobenzoate; prednisolone sodium phosphate; prednisolone steaglate; prednisolone tebutate; prednisolone tetrahydrophthalate; prednisone; prednival; prednylidene; pregnenolone; procinonide; tralonide; progesterone; promegestone; rhapontisterone; rimexolone; roxibolone; rubrosterone; stizophyllin; tixocortol; topterone; triamcinolone; triamcinolone acetonide; triamcinolone acetonide 21-palmitate; triamcinolone diacetate; triamcinolone hexacetonide; trimegestone; turkesterone; and wortmannin.

Standard recommended dosages for corticosteroids are provided, e.g., in the Merck Manual of Diagnosis & Therapy (17th Ed. MH Beers et al., Merck & Co.) and Physicians' Desk Reference 2003 (57th Ed. Medical Economics Staff et al., Medical Economics Co., 2002). In one embodiment, the dosage of corticosteroid administered is a dosage equivalent to a prednisolone dosage, as defined herein. For example, a low dosage of a corticosteroid may be considered as the dosage equivalent to a low dosage of prednisolone. Two or more corticosteroids can be administered in the same treatment. This combination can be especially useful for situations in which the dominant menstrually related symptom experienced by the subject is swelling.

### Antidepressants

If desired, the ketamine, or a pharmaceutically acceptable salt thereof, may be administered in conjunction with one or more antidepressants, such as fluoxetine, duloxetine, bupropion, citalopram, escitalopram, paroxetine, lorazepam, fluvoxamine, sertraline, desvenlafaxine, milnacipran, venlafaxine, amitriptyline, nortriptyline, desipramine, alprazolam, agomelatine, etoperidone, or phenelzine. Two or more antidepressants can be administered in the same treatment. This combination can be especially useful for situations in which the dominant menstrually related symptom experienced by the subject is anxiety or dysphoria.

### Examples

The following examples are put forth to provide those of ordinary skill in the art with a description of how the compositions and methods described herein may be used, made, and evaluated, and are intended to be purely exemplary of the invention and are not intended to limit the scope of the invention which is defined in the claims.

### Example 1. Sublingual Ketamine Lozenges for the Treatment of PMS.

OVERVIEW: 9 women completed a 2 month or more evaluation of sublingual ketamine lozenges' effectiveness for symptoms of menstrual related pain and emotion. Using a validated instrument-the Daily Record of Severity of Problems-participating women were asked to enter data for the severity of symptoms on symptomatic days of their menstrual cycle. This was recorded for each use of ketamine as well as their providing qualitative commentary.

METHOD: 50 mg ketamine (ketamine racemate) sublingual lozenges, standardized by independent assay, were distributed by the Investigator to each subject. Both oral dissolving tablets and standard waxy lozenges were provided to determine if there were differences between the two identically dosed preparations. Women enrolled in the study were of child bearing age and having mostly regular menstrual cycles. Several of the women were not naive to ketamine use in their past. Subjects were asked to rate the effectiveness of their use of the lozenges and the duration of effect for both physical and emotional symptoms separately. All women were prepared for ketamine use personally by the Investigator and signed an informed consent document. Blood pressures and heart rates were taken to assure basic safety in terms of cardiac status. Concurrent medications and illnesses were evaluated personally by the physician and through a thorough intake form reviewed with the subjects.

The oral rapidly dissolving tablets take less than a minute to two minutes to dissolve in the mouth and onset of effects (7-10 minutes) tends to be quicker due to more rapid availability for mucosal absorption. The sublingual lozenges are of a waxy nature and tend to take 5 minutes to dissolve with onset of effect at 15 minutes or longer. Subjects are instructed to hold both varieties of lozenges for 10 minutes in the mouth to expose the oral mucosa to the activated saliva and on a signal to swallow. Lozenges/ODTs are our method of choice for ketamine for this use because of the difficulty of abuse and their safety.

Preparation of the lozenges: Melt the polyglycol base on hot plate at ca. 100°C while stirring. Using a mortar and pestle, triturate the racemic ketamine hydrochloride and silica gel to a fine powder. Sift the powder into the melted base using a strainer and stir until an evenly dispersed melt is formed. Add a flavoring agent into the melt and mix well. Pour the contents into molds and allow the melt to congeal at room temperature.

Preparation of the rapidly dissolving tablets: Triturate racemic ketamine hydrochloride, steviol glycosides 95%, acesulfame potassium, flavor powder and RDT-Plus^{™} base (PCCA^{®}) together in a mortar and pestle to reduce particle size and obtain a uniform mixture. Into a rapid dissolve tablet (RDT) mold, pour approximately the formula powder, pressing the powder into the mold. Bake the mold in an oven at 110°C for 15 minutes. Remove the RDT Mold from the oven. Remove the tablets from the RDT mold by inverting the mold and tapping. Allow the ROTs to cool for an additional 10 minutes prior to packaging.

Subjects were instructed to assess the efficacy of the lozenges beginning with a half dose of 25 mgs obtained by dividing the lozenges/ODTs. Progression to 50 mg ketamine racemate with a request to not exceed 100 mg in a day was the dosing protocol. Dosing was designed to affect the activities of daily living as little as possible, while specifying that activities such as driving or operating machinery be put off for several hours until completely back to baseline.

Subjects were provided a limited number of doses (8 ea) 50 mg lozenges/ODTs and were advised to request more if necessary with supporting rational including continuing for additional cycles. Subjects were advised to:
"Take your dose the next day if symptoms continue and every day until cessation of symptoms- use the lowest dose you feel is effective. Use your judgement with respect to your severity of symptoms in terms of continuing to use ketamine in succeeding days. In other words, if your symptoms are tolerable, do not use the ketamine lozenges. If symptom free the following day, wait and take next dose only if symptom(s) returns."

### Consultation with the Investigators by phone or email was a component part of the study

RESULTS: All 9 subjects reported emotional symptom improvement during the symptomatic days of their cycles. Three subjects reported sufficient improvement with a 25 mg dose. One subject reported that a 50 mg dose interfered with her functionality. All subjects reported a duration of effect lasting for days, or more, beyond the time of ketamine administration. Several used ketamine at night so as not to interfere with functionality. The anesthetic effects of sublingual ketamine lasted up to 2 hours in duration with the first hour (more or less) being the intense portion of the experience. Three subjects preferred the 100 mg dose, which was reported to give substantial relief of PMS symptoms. Five subjects indicated their preference for the 50 mg dose.

All subjects indicated a desire to continue ketamine use and two subjects continued ketamine treatment over 3 and 4 menstrual cycles, and reported over this treatment period.

In terms of physical symptoms, breast tenderness and cramping tended to be positively affected in three subjects, but not in six others. Two subjects reported improvement of headaches. Two subjects reported relief of their physical symptoms for 5 days until menses began following ketamine doses at 50 mg and 100 mg, respectively.

In terms of adverse effects, one subject reported transient nausea on 2 of her 11 sessions with ketamine dosing at 25 mg and 50 mg. There were no reports of negative after effects.

The sample size for this study was too small to differentiate the benefits of lozenges versus ODTs.

Subject comments included the following: Amazing; a godsend during worst period; more stable emotionally; relief of irritability, anxiety and hopelessness; overall feeling of wellness before my period; manageable; relief of anxiety, anger, rage, and dysphoria; the evening treatment reset me; mental clarity; decreased agitation; greater clarity and centering.

DISCUSSION: Ketamine lozenge/ODT effectiveness was perceived to be favorable predominantly for emotional symptoms with a tendency to persevere during subsequent days of the menstrual cycle otherwise expected to be symptomatic. The efficacious dosing level was 25-100 mg. This indicates that a standardized preparation of 50 mg in this format would be the desired dose allowing for half or two dose regimens depending on the particular individual's response. Given the small sample size, the study can only suggest that some women would be benefitted for some of the physical symptoms of the menstrual cycle.

Generalizing from the study, ketamine appears to be an effective medicine for ameliorating menstrual cycle symptoms. Limitations may include interference with some functionality. Other low dose regimens will be considered including nasal insufflation in a controlled dose format in an effort to balance effectiveness with capacity to perform daily activities.

Given the pervasiveness of menstrual symptoms impact on women's lives, this small study as a proof of concept supports the safe use of ketamine for the treatment of menstrually related symptoms, including symptoms associated with PMS, PMDD, menopause, and perimenopause.

### Example 2. Lozenges Containing 6-Hydroxynorketamine for the Treatment of Subclinical Menstrually Related Symptoms.

Lozenges containing isomerically pure (2R,6R)-6-hydroxynorketamine hydrochloride (50 mg) are prepared by mixing one cup (240 grams) of sugar, ⅓ cup (81 cc) of light corn syrup, and slightly more than 1 cup (240 ml) of water. The mixture is heated to a temperature of at least 285° F., taking care to avoid stirring the mixture at temperatures greater than 200° F. to prevent uncontrolled crystallization of the sugar mixture. The mixture is allowed to cool to 260° F., and 4 ml of a flavoring agent and ⅛ teaspoon (0.625 cc) of citric acid are added, followed by the addition of 900 mg (2R,6R)-6-hydroxynorketamine hydrochloride and 1800 mg sodium phosphate dibasic. These ingredients are stirred thoroughly, and the resulting mixture is poured into molds which have been sprayed with an anti-stick coating and cooled to produce 50 mg lozenges. The lozenges are scored to easily permit divided (e.g., half) dosing. The ODTs are provided to subjects suffering from subclinical menstrually related symptoms. The subjects can administer from ½ to 2 tablets once, twice, or three times daily upon the appearance of the symptoms. The subjects with subclinical menstrually related symptoms experience a reduction in anxiety, anger, and/or dysphoria. Furthermore, the subjects can experience an increase in mental clarity.

### Example 3. Intranasal S-(+)-ketamine for the Treatment of PMDD.

Enantiomerically pure S-(+)-ketamine hydrochloride (eq. 150 mg/mL) and a penetration enhancer (10 mg/mL) (e.g., tauroursodeoxycholic acid) are mixed with water and the pH of the resulting mixture is adjusted with 1N NaOH to approximately pH 4.51. Subjects suffering from premenstrual dysphoric disorder (PMDD) are provided with a nasal spraying configured to deliver a dose of 0.2 ml per spray to a nostril of the subject (30 mg per dose). The subjects can administer from 1 to 4 intranasal doses daily upon the appearance of symptoms. The doses can be administered at night so as not to interfere with the functionality of the subject. The subjects with PMDD experience a reduction in anxiety, anger, and/or dysphoria. Furthermore, the subjects can experience an increase in mental clarity.

## Claims

1. Ketamine or a pharmaceutically acceptable salt thereof, for use in a method of treating a menstrually related symptom in a subject in need threreof, wherein said menstrually related symptom is selected from mood swings, dysphoria, negativism, diminished mental capabilities, anxiety, irritability, and anger; wherein the symptom is caused by premenstrual syndrome (PMS) or premenstrual dysphoric disorder (PMDD), and wherein an average daily dose of from 1 mg to 500 mg of ketamine, or a pharmaceutically acceptable salt thereof, is administered to the subject.

2. Ketamine or a pharmaceutically acceptable salt thereof, for the use according to claim 1, wherein the menstrually related symptom is caused by PMS.

3. Ketamine or a pharmaceutically acceptable salt thereof, for the use according to claim 1, wherein the menstrually related symptom is caused by PMDD.

4. Ketamine or a pharmaceutically acceptable salt thereof, for the use according to any one of claims 1-3, wherein an average daily dose of from 10 mg to 200 mg of enantiomerically pure S-(+)-ketamine, or a pharmaceutically acceptable salt thereof, is administered to the subject.

5. Ketamine or a pharmaceutically acceptable salt thereof, for the use according to any one of claims 1-3, wherein an average daily dose of from 10 mg to 200 mg of enantiomerically pure R-(-)-ketamine, or a pharmaceutically acceptable salt thereof, is administered to the subject.

6. Ketamine or a pharmaceutically acceptable salt thereof, for the use according to any one of claims 1-3 , wherein an average daily dose of from 10 mg to 200 mg of racemic ketamine, or a pharmaceutically acceptable salt thereof, is administered to the subject.

7. Ketamine or a pharmaceutically acceptable salt thereof, for the use according claim 1, wherein the ketamine, or a pharmaceutically acceptable salt thereof, is administered sublingually.

8. Ketamine or a pharmaceutically acceptable salt thereof, for the use according to claim 1, wherein the menstrually related symptom is mood swings and the treatment ameliorates mood swings.

9. Ketamine, or a pharmaceutically acceptable salt thereof, for the use according to claim 1, wherein the menstrually related symptom is irritability.

10. Ketamine or a pharmaceutically acceptable salt thereof, for the use according to claim 1, wherein the menstrually related symptom is anger.

## Patentansprüche

1. Ketamin oder ein pharmazeutisch verträgliches Salz davon zur Verwendung in einem Verfahren zur Behandlung eines menstruationsbedingten Symptoms bei einem Individuum, das dessen bedarf, wobei das menstruationsbedingte Symptom ausgewählt ist aus Stimmungsschwankungen, Dysphorie, Negativismus, eingeschränkten mentalen Fähigkeiten, Angstzuständen, Reizbarkeit und Aggressivität; wobei das Symptom durch ein prämenstruelles Syndrom (PMS) oder eine prämenstruelle dysphorische Störung (PMDS) verursacht wird, und wobei dem Individuum eine durchschnittliche tägliche Dosis von 1 mg bis 500 mg von Ketamin oder einem pharmazeutisch verträglichen Salz davon verabreicht wird.

2. Ketamin oder ein pharmazeutisch verträgliches Salz davon zur Verwendung nach Anspruch 1, wobei das menstruationsbedingte Symptom durch PMS verursacht wird.

3. Ketamin oder ein pharmazeutisch verträgliches Salz davon zur Verwendung nach Anspruch 1, wobei das menstruationsbedingte Symptom durch PMDS verursacht wird.

4. Ketamin oder ein pharmazeutisch verträgliches Salz davon zur Verwendung nach einem der Ansprüche 1 bis 3, wobei dem Individuum eine durchschnittliche tägliche Dosis von 10 mg bis 200 mg von enantiomer reinem S-(+)-Ketamin oder einem pharmazeutisch verträglichen Salz davon verabreicht wird.

5. Ketamin oder ein pharmazeutisch verträgliches Salz davon zur Verwendung nach einem der Ansprüche 1 bis 3, wobei dem Individuum eine durchschnittliche tägliche Dosis von 10 mg bis 200 mg von enantiomer reinem R-(-)-Ketamin oder einem pharmazeutisch verträglichen Salz davon verabreicht wird.

6. Ketamin oder ein pharmazeutisch verträgliches Salz davon zur Verwendung nach einem der Ansprüche 1 bis 3, wobei dem Individuum eine durchschnittliche tägliche Dosis von 10 mg bis 200 mg von racemischem Ketamin oder einem pharmazeutisch verträglichen Salz davon verabreicht wird.

7. Ketamin oder ein pharmazeutisch verträgliches Salz davon zur Verwendung nach Anspruch 1, wobei das Ketamin oder ein pharmazeutisch verträgliches Salz davon sublingual verabreicht wird.

8. Ketamin oder ein pharmazeutisch verträgliches Salz davon zur Verwendung nach Anspruch 1, wobei das menstruationsbedingte Symptom sich durch Stimmungsschwankungen zeigt, und die Behandlung die Stimmungsschwankungen verbessert.

9. Ketamin oder ein pharmazeutisch verträgliches Salz davon zur Verwendung nach Anspruch 1, wobei das menstruationsbedingte Symptom sich durch Reizbarkeit zeigt.

10. Ketamin oder ein pharmazeutisch verträgliches Salz davon zur Verwendung nach Anspruch 1, wobei das menstruationsbedingte Symptom sich durch Aggressivität zeigt.

## Revendications

1. Kétamine ou un de ses sels pharmaceutiquement acceptables, destinée à être utilisée dans un procédé de traitement de symptôme associé aux menstrues chez un sujet en ayant besoin, dans laquelle ledit symptôme associé aux menstrues est choisi parmi les sautes d'humeur, la dysphorie, le négativisme, des capacités mentales diminuées, l'anxiété, l'irritabilité et la colère ; le symptôme étant provoqué par le syndrome prémenstruel (PMS) ou le trouble dysphorique prémenstruel (PMDD) et une dose quotidienne moyenne de 1 mg à 500 mg de kétamine, ou d'un de ses sels pharmaceutiquement acceptables, étant administrée au sujet.

2. Kétamine ou un de ses sels pharmaceutiquement acceptables, destinée à être utilisée selon la revendication 1, dans laquelle le symptôme associé aux menstrues est provoqué par le PMS.

3. Kétamine ou un de ses sels pharmaceutiquement acceptables, destinée à être utilisée selon la revendication 1, dans laquelle le symptôme associé aux menstrues est provoqué par le PMDD.

4. Kétamine ou un de ses sels pharmaceutiquement acceptables, destinée à être utilisée selon l'une quelconque des revendications 1 à 3, dans laquelle une dose quotidienne moyenne de 10 mg à 200 mg de S-(+)-kétamine énantiomériquement pure ou un de ses sels pharmaceutiquement acceptables, est administrée au sujet.

5. Kétamine ou un de ses sels pharmaceutiquement acceptables, destinée à être utilisée selon l'une quelconque des revendications 1 à 3, dans laquelle une dose quotidienne moyenne de 10 mg à 200 mg de R-(-)-kétamine énantiomériquement pure ou un de ses sels pharmaceutiquement acceptables, est administrée au sujet.

6. Kétamine ou un de ses sels pharmaceutiquement acceptables, destinée à être utilisée selon l'une quelconque des revendications 1 à 3, dans laquelle une dose quotidienne moyenne de 10 à 200 mg de kétamine racémique ou un de ses sels pharmaceutiquement acceptables, est administrée au sujet.

7. Kétamine ou un de ses sels pharmaceutiquement acceptables, destinée à être utilisée selon la revendication 1, dans laquelle la kétamine ou un de ses sels pharmaceutiquement acceptables, est administrée sublingualement.

8. Kétamine ou un de ses sels pharmaceutiquement acceptables, destinée à être utilisée selon la revendication 1, dans laquelle le symptôme associé aux menstrues est les sautes d'humeur et le traitement améliore les sautes d'humeur.

9. Kétamine ou un de ses sels pharmaceutiquement acceptables, destinée à être utilisée selon la revendication 1, dans laquelle le symptôme associé aux menstrues est l'irritabilité.

10. Kétamine ou un de ses sels pharmaceutiquement acceptables, destinée à être utilisée selon la revendication 1, dans laquelle le symptôme associé aux menstrues est la colère.
